(19) Europäisches Patentamt
European Patent Office
Office européen des brevets

(11) Veröffentlichungsnummer : **0 471 388 B1**

(12) # EUROPÄISCHE PATENTSCHRIFT

(45) Veröffentlichungstag der Patentschrift : **14.07.93 Patentblatt 93/28**

(51) Int. Cl.$^5$ : **A61K 31/55**

(21) Anmeldenummer : **91118481.0**

(22) Anmeldetag : **15.10.88**

(54) **Mittel zur Behandlung der Herzinsuffizienz.**

Die Akte enthält technische Angaben, die nach dem Eingang der Anmeldung eingereicht wurden und die nicht in dieser Patentschrift enthalten sind.

(30) Priorität : **30.10.87 DE 3736866**

(43) Veröffentlichungstag der Anmeldung : **19.02.92 Patentblatt 92/08**

(45) Bekanntmachung des Hinweises auf die Patenterteilung : **14.07.93 Patentblatt 93/28**

(84) Benannte Vertragsstaaten : **AT BE CH DE FR GB IT LI LU NL SE**

(56) Entgegenhaltungen :
**EP-A- 065 229**
**BR. J. PHARMACOL., Band 94, Nr. 1, Mai 1988, Seiten 55-64; G. KRUMPL et al.: "Comparison of the haemodynamic effects of the selective bradycardic agent UL-FS 49, with those of propranolol during treadmill exercise in dogs"**
**EUROPEAN HEART JOURNAL, Band 8, (Supplement L), Mai 1987, Seiten 53-59, The European Society of Cardiology; G. RABERGER et al.: "Effects of specific bradycardic agents on exercise-induced regional myocardial dysfunction in dogs"**
**NAUNYN-SCHMIEDEBERG'S ARCHIVES OF PHARMACOLOGY, Band 334, 1986, Seiten 540-543, Springer-Verlag; G. KRUMPL et al.: "Can exercise-induced regional contractile dysfunction be prevented by selective bradycardic agents?"**
**JOURNAL OF CARDIOVASCULAR PHARMACOLOGY, Band 8, Nr. 4, 1986, Seiten 791-797, Raven Press, New York, US; C. LILLIE et al.: "Investigations into the bradycardic effects of UL-FS 49 (1,3,4,5-tetrahydro-7,8-dimethoxy-3-[3-[[2-(3,4-dimethoxyphenyl))ethyl]methylimino]propyl]-2H-3-benzazepin-2-on-hydrochloride) in isolated Guinea pig atria"**

(56) Entgegenhaltungen :
**IDEM**
**EUROPEAN HEART JOURNAL, Band 8, (Supplement L), 1987, Seiten 61-68, The European Society of Cardiology; B.D. GUTH et al.: "Role of heart rate reduction in the treatment of exercise-induced myocardial ischaemia"**

(60) Veröffentlichungsnummer der früheren Anmeldung nach Art. 76 EPü : **0 313 945**

(73) Patentinhaber : **Dr. Karl Thomae GmbH**
**Postfach 1755**
**W-7950 Biberach 1 (DE)**

(72) Erfinder : **Rose, Peter, Dr.**
**Amriswilstrasse 7**
**W-7950 Biberach 1 (DE)**

EP 0 471 388 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

In der EP-B-0.065.229 wird die Verbindung der Formel

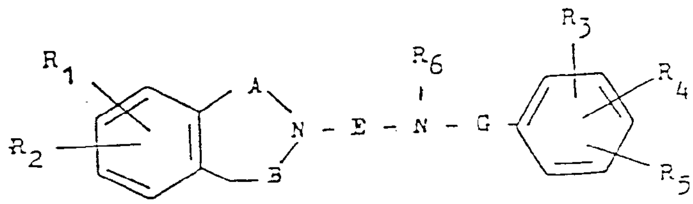

(I),

in der

A eine $-CH_2-CH_2-$, -CH=CH-,

-NH-CO-, $-CH_2-CO-$
5 5

oder

R7
-C =N-Gruppe
5

in der $R_7$ eine Alkylgruppe mit 1 bis 3 Kohlenstoffatomen darstellt, und B die Methylen- oder Carbonylgruppe oder

A die -CO-CO-Gruppe und B die Methylengruppe,

E eine gegebenenfalls durch eine Alkylgruppe mit 1 bis 3 Kohlenstoffatomen substitutierte Ethylen-, n-Propylen- oder n-Butylengruppe, eine durch eine Hydroxygruppe in 2-Stellung substituierte n-Propylengruppe oder eine durch eine Hydroxygruppe in 2- oder 3-Stellung substituierte n-Butylengruppe,

G eine gegebenenfalls durch eine Alkylgruppe mit 1 bis 3 Kohlenstoffatomen substituierte Methylen- oder Ethylengruppe,

$R_1$ und $R_2$, die gleich oder verschieden sein können, Hydroxygruppen, Alkyl-, Alkoxy- oder Phenylalkoxygruppen, in denen der Alkylteil jeweils 1 bis 3 Kohlenstoffatome enthalten kann, oder einer der Reste $R_1$ oder $R_2$ auch ein Wasserstoffatom oder $R_1$ zusammen mit $R_2$ eine Alkylendioxy-gruppe mit 1 oder 2 Kohlenstoffatomen,

$R_3$ und $R_4$, die gleich oder verschieden sein können, Wasserstoff- oder Halogenatome, Hydroxygruppen, Alkyl- oder Alkoxygruppen mit jeweils 1 bis 4 Kohlenstoffatomen, Trifluormethyl- oder Cyangruppen oder einer der Reste $R_3$ oder $R_4$ auch eine Nitrogruppe oder $R_3$ zusammen mit $R_4$ eine Alkylendioxygruppe mit 1 oder 2 Kohlenstoffatomen,

$R_5$ ein Wasserstoffatom, eine Alkyl-, Hydroxy-, Alkoxy-, Amino-, Alkylamino-, Dialkylamino-, Alkanoylamino-, Alkoxycarbonylamino- oder Bis(alkoxycarbonyl)aminogruppe, in denen der Alkylteil jeweils 1 bis 3 Kohlenstoffatome enthalten kann, oder eine durch eine Trifluormethylgruppe substitutierte Methyalmino- oder Ethylaminogruppe, und

$R_5$ ein Wasserstoffatom, eine Alkyl-, Phenylalkyl, Alkanoyl- oder Alkoxycarbonylgruppe, in denen der Alkylteil jeweils 1 bis 3 Kohlenstoffatome enthalten kann, oder eine Alkylengruppe mit 3 bis 5 Kohlenstoffatomen bedeuten, und deren Säureadditionssalze, insbesondere deren physiologisch verträgliche Säureadditionssalze mit anorganischen oder organischen Säuren, beschrieben, wobei festgestellt wird, daß dieser Verbindung und deren Säureadditionssalzen wertvolle pharmakologische Eigenschaften zukommen, z.B. eine lang anhaltende herzfrequenzsenkende Wirkung sowie eine Herabsetzung des $O_2$-Bedarfs des Herzens.

Diese Verbindungen wurden daher als neuartige Substanzen entwickelt, welche die Herzfrequenz über einen direkten Angriff am Sinusknoten senken. Hierdurch kommen den Substanzen antiischämische Eigenschaften zu, d.h. neben einer Senkung des myokardialen Energieverbrauches durch die Herzfrequenz wird gleichzeitig das Sauerstoffangebot über eine Verlängerung der Diastole erhöht. pharmakologischen Untersuchungen zufolge beeinflussen die Substanzen weder die Inotropie noch den Blutdruck oder die AV-Überleitung. Tierexperimentell konnte ein zusätzlicher sog. myokardprotektiver Effekt nachgewiesen werden. Somit erscheinen die Substanzen geeignet für die Therapie der stabilen koronaren Herzkrankheit.

Lillie et al. stellen in dem Journal of Cardiavascular pharmacology of 1986, 8(4), p.971-7 eine Untersuchung der bradycardischen Wirkung von UL-FS 59 an isolierten Meerschweinchenarterien dar. Sie führen aus, daß UL-FS 49 spezifisch den Herzschlag reduziert, nicht aber die Kontratilität während der Stimulation der β-Adrenoceptoren und weiter, daß UL-FS 49 ziemlich stark den spezifischen Bradikardiken Alinidine und Falipamil ähneln.

Guth et al. stellen im European Heart Journal (1987), 8 (Suppl. L), S. 53-59, die günstige Mitwirkung von UL-FS 49 in der Behandlung von belastungsinduzierter Myokardischämie dar.

Gegenstand der Erfindung ist die Verwendung der Verbindungen der allgemeinen Formel I und deren Säureadditionssalze, insbesondere deren physiologisch verträglichen Säureadditionssalze mit anorganischen oder organischen Säure, zur Herstellung eines pharmazeutischen Präparates zur Behandlung von Herzinsuffizienz.

Vorzugsweise verwendet man 1,3,4,5-tetrahydro-7,8-dimethoxy-3-[3-[[2-(3,4-dimethoxyphenyl)ethyl] methyl]amino]propyl]-2H-3-benzazepin-2-on und deren physiologisch verträglichen Säureadditionssalze, insbesondere deren Hydrochlorid, das im folgenden als UL-FS 49 CL bezeichnet wird.

Es wird eine Tagesdosis von zweimal täglich 0.015 bis 0.2 mg/kg Körpergewicht, vorzugsweise 0.0175 mg/kg bis 0.175 mg/kg Körpergewicht erwartet, z.B. zwischen 1.25 und 7.5 mg/ Mensch oral zweimal täglich bewegen. Die genaue Dosis wird offensichtlich vom Zustand des Patienten und der entsprechenden Prognose der Krankheit abhängen und könnte von der oben angegeben Dosierung abweichen.

Zur medizinischen Anwendung läßt sich das Arzneimittel mit Hilfe von üblichen galenischen Hilfsstoffen wie z.B. Milchzucker, Mannit, Rohrzucker, mikrokristalline Zellulose, Magnesiumstearat, Polyvinylpyrollidon, Zitronensäure, Weinsäure, Wasser, Wasser/Äthanol, Wasser/Glycerin, Wasser/Sorbit, Wasser/Polyethylenglykol, Propylenglykol, Carboxymethylcellulose oder fetthaltigen Substanzen wie Hartfett oder deren geeigneten Gemischen in den üblichen Zubereitungen wie Tabletten, Dragées, Kapseln, Pulver, Suspensionen, Tropfen, Ampullen, Säfte oder Zäpfchen herstellen.

Die Wirkung der Verbindungen wurde wie folgt festgestellt.

In einer doppelblinden, placebokontrollierten klinischen Prüfung des antianginösen Effekts von UL-FS 49 CL bei Patienten mit koronarer Herzkrankheit wurden insgesamt 60 Patienten in einer Studie aufgenommen. Alle Patienten waren bei Studienbeginn unbehandelt. In der 1. Woche (Tag -7 bis Tag -1) erhielten alle 60 Patienten Placebo. Während den darauffolgenden 4 Wochen wurden die 60 Patienten in 3 Gruppen zu je 20 Patienten eingeteilt. Die 1. Gruppe erhielt während dieser Zeit weiterhin Placebo. Die 2. Gruppe 2x 2.5 mg des Wirkstoffs und die 3. Gruppe 2x 7.5 mg des Wirkstoffs oral täglich (siehe Abb. 1).

An den Tagen -7, +1 und +28 wurden folgende Untersuchungen durchgeführt:

Klinische Untersuchung, Labor sowie EKG, Herzfrequenz und Blutdruck in Ruhe und unter Belastung.

In einer Zwischenauswertung waren die vollständigen Daten von 48 Patienten verwendbar. Neben der Senkung der Herzfrequenz und Erhöhung der Belastungsdauer konnte überraschenderweise eine deutliche Blutdrucksenkung festgestellt werden. Die nachfolgenden Daten beziehen sich jeweils auf die Zeitpunkte 2 Stunden nach Applikation.

In der Placebo-Gruppe (n=17) blieben sowohl der systolische als auch der diastolische Blutdruck praktisch unverändert (Tab. 1, 2 und Abb. 2, 3). In der 2.5-mg-Gruppe (n=15) fiel der systolische Blutdruck von 157 ± 10 mm Hg (Tag -7) auf 142 ± 16 mm Hg (Tag +28) mit $p<0.001$ (Tab. 1, Abb. 2). Im gleichen Zeitraum fiel der diastolische Blutdruck von 95 ± 8 mm Hg auf 86 ± 6 mm Hg mit ebenfalls $p<0.001$ (Tab. 2, Abb. 3). In der 7.5-mg-Gruppe (n=16) wurde der systolische Blutdruck von 154 ± 14 mm HG (Tag -7) auf 137 ± 13 mm Hg (Tag +28) mit $p<0.001$ gesenkt (Tab. 1, Abb. 2). Der diastolische Blutdruck reduzierte sich in dieser Behandlungsgruppe von 93 ± 8 mm Hg auf 86 ±6 mm Hg mit $p<0.01$ (Tab. 2, Abb. 3).

Weder pharmakologisch noch klinisch-pharmakologisch wurden bisher blutdrucksenkende Effekte von UL-FS 49 CL und deren Säureadditionssalzen beschrieben. Physiologisch gesehen ist bei einer Senkung der Herzfrequenz, wenn überhaupt, dann mit einer Erhöhung des systolischen Blutdruckes und gleichzeitig geringer Absenkung des diastolischen Blutdruckes zu rechnen. Der arterielle Mitteldruck müßte hierbei jedoch unverändert bleiben.

In der vorliegenden Studie konnte eindeutig ein statistisch signifikanter und klinisch relevanter Abfall von systolischem und diastolischem Blutdruck nachgewiesen werden.

Auf Grund dieser neuen Befunde eignen sich die vorliegenden Verbindungen, insbesondere UL-FS 49 CL, zur Behandlung der Herzinsuffizienz.

Akute Toxizität

Die akute Toxizität von UL-FS 49 CL wurde an Mäusen, Ratten und Kaninchen nach den üblichen Methoden bestimmt. Aus dem Prozentsatz der Tiere, die nach den verschiedenen Dosen innerhalb von 14 Tagen verstarben, wurde nach Litchfield und Wilcoxon (J. Pharmacol. exp. Ther., 96, 99 (1949)) die $LD_{50}$ berechnet.

| Tierart | $LD_{50}$ mg/kg | |
|---|---|---|
| | oral | parenteral |
| Maus | 1.350 | i.v. 89 |
| Ratte | 479 | - |
| Kaninchen | - | i.v. 45 |

BEISPIEL 1

Filmtabletten zu 1,25 mg

A. Tablettenkern:

Zusammensetzung:

| | |
|---|---|
| (01) UL-FS 49 CL | 1.25 mg |
| (02) Lactose x $H_2O$ | 30.00 mg |
| (03) Maisstärke | 16.00 mg |
| (04) Kollidon 25 | 2.50 mg |
| (05) Magnesiumstearat | 0.25 mg |
| | 50.00 mg |

Herstellung:

Der Wirkstoff wird mit 5 mg (02) gründlich verrieben. Die Verreibung wird mit (03) und dem Rest von (02) gemischt und mit einer wäßrigen Lösung von (04) gleichmässig befeuchtet. Die feuchte Masse wird durch ein Sieb von 1,5 mm Maschenweite gegeben, in einem Umluftschrank getrocknet, erneut gesiebt (1.0 mm Maschenweite) und mit (05) vermischt. Aus dieser Mischung werden bikonvex gewölbte Tabletten von 5 mm Durchmesser und 50 mg Gewicht auf einer Tablettiermaschine hergestellt.

B. Filmüberzug

Zusammensetzung:

| | | |
|---|---|---|
| (01) | Hydroxypropylmethylcellulose | 1.5 mg |
| (02) | Polyethylenglykol 6000 | 0.3 mg |

Herstellung:

Die Filmbestandteile (01) und (02) werden in Wasser gelöst und mit Hilfe einer Zweistoffdüse auf die Tablettenkerne aufgesprüht.

Beschreibung einer Filmtablette

| | |
|---|---|
| Aussehen: | weiß, rund, bikonvex |
| Durchmesser: | ca. 5.1 mm |
| Gewicht: | ca. 51.8 mg |

BEISPIEL 2

Drageés zu 1,25 mg

A. Drageékern

vgl. Beispiel 1 A.

B. Drageéüberzug

Erfolgt in üblicher Weise mit einer zuckerhaltigen Dragiersuspension in einem gebräuchlichen Dragierkessel.

Beschreibung eines Drageés

| | |
|---|---|
| Aussehen: | weiß, rund, bikonvex |
| Durchmesser: | ca. 6 mm |
| Gewicht: | ca. 70 mg |

BEISPIEL 3

Kapsel mit 7.5 mg UL-FS 49 CL

Zusammensetzung:

1 Kapsel enthält:

| | |
|---|---|
| UL-FS 49 CL | 7.5000 mg |
| Maisstärke getr. | 85.7500 mg |
| Lactose x $1H_2O$ | 85.7500 mg |
| Magnesiumstearat | 1.0000 mg |
| | 180.0000 mg |

Kapselhülle:

| | |
|---|---|
| Hartgelatine-Kapseln Nr. 3 lederfarben | 50.0000 mg |
| | 230.0000 mg |

Herstellungsverfahren:

Wirkstoff, Maisstärke und Lactose werden gemischt, mit Granulierflüssigkeit (Ethanol/Wasser 1+1 G/G) durchfeuchtet und gesiebt.

Nach Trocknung bei 50°C wird das Granulat nochmals gesiebt und anschließend Magnesiumstearat zugegeben.

Das komplette Kapselgranulat wird homogen gemischt und auf einer geeigneten Kapselmaschine in Hartgelatinekapseln abgefüllt.

BEISPIEL 4

Ampullen mit 5 mg UL-FS 49 CL

| Zusammensetzung: | | mg/5 ml |
|---|---|---|
| UL-FS 49 CL | | 5.000 mg |
| Citronensäure x $H_2O$ | | 2.000 mg |
| 1N NaOH ad pH 6.0 | ca. | 0.025 ml |
| NaCl | | 46.500 mg |
| $H_2O$ f. Injektionszwecke | ad | 5.000 ml |

Herstellungsverfahren:

In Wasser für Injektionszwecke werden Citronensäure, Wirkstoff und Natriumchlorid bei Raumtemperatur gelöst. In 1N NaOH wird auf pH 6 eingestellt und mit Wasser für Injektionszwecke auf das Ansatzgewicht aufgefüllt. Nach Sterilfiltration wird die Lösung in Spießampullen abgefüllt. Die gefüllten Ampullen werden 20 Minuten bei 121°C autoklaviert.

BEISPIEL 5

Suppositorien mit 10 mg UL-FS 49 CL

Zusammensetzung:

| | |
|---|---|
| UL-FS 49 CL | 0.010 g |
| Hartfett (z.B. Witepsol H 19 und Witepsol W 45) | 1.690 g |
| | 1.700 g |

Herstellungsverfahren:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

Ein Zäpfchen zu 1.7 g enthält 10 mg UL-FS 49 CL.

BEISPIEL 6

Tropfenlösung, enthaltend 5 mg/l ml/20 Tropfen UL-FS 49 CL

Zusammensetzung:

| | | |
|---|---|---|
| UL-FS 49 CL | | 0.50 g |
| Hydroxyethylcellulose | | 0.15 g |
| Weinsäure | | 0.10 g |
| Sorbitlösung 70 % Trockensubstanz | | 30.00 g |
| Glycerin | | 10.00 g |
| Benzoesäure | | 0.15 g |
| Dest. Wasser | ad | 100.00 ml |

Herstellungsverfahren:

Dest. Wasser wird auf 70°C erhitzt. Hierin wird unter Rühren Hydroxyethylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung der Lösung unter Rühren evakuiert.

1 ml, entsprechend 20 Tropfen der Tropfenlösung, enthalten 5,0 mg UL-FS 49 CL.

BEISPIEL 7

Saft mit 2.5 mg UL-EFS 49 CL / 5 ml

Zusammensetzung:

| | | |
|---|---|---|
| UL-FS 49 CL | | 0.05 g |
| Carboxymethylcellulose | | 0.10 g |
| p-Hydroxybenzoesäuremethylester | | 0.05 g |
| p-Hydroxybenzoesäurepropylester | | 0.03 g |
| Saccarose | | 10.00 g |
| Glycerin | | 5.00 g |
| Sorbitlösung 70 % | | 20.00 g |
| Aroma | | 0.30 g |
| Wasser, dest. | ad | 100.00 ml |

Herstellungsverfahren:

Dest. Wasser wird auf 70°C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und gelöst. Nach Zugabe und Lösung der Saccarose, der Sorbitlösung und

des Aromas wird der Saft zur Entlüftung unter Rühren evakuiert.

5 ml Saft enthalten 2,5 mg UL-FS 49 Cl.

Tabelle 1

Blutdruck systolisch

| | Placebo | 2 x 2,5 mg UL-FS 49 CL | 2 x 7,5 mg UL-FS 49 CL |
|---|---|---|---|
| Tag - 7 | 148 ± 18 | 157 ± 10 | 154 ± 14 |
| Tag + 1 | 147 ± 13 | 145 ± 18 | 146 ± 16 |
| Tag + 28 | 150 ± 14 | 142 ± 16 | 137 ± 13 |

Tabelle 2

Blutdruck diastolisch

| | Placebo | 2 x 2,5 mg UL-FS 49 CL | 2 x 7,5 mg UL-FS 49 CL |
|---|---|---|---|
| Tag - 7 | 91 ± 7 | 95 ± 8 | 93 ± 8 |
| Tag + 1 | 90 ± 7 | 90 ± 6 | 91 ± 7 |
| Tag + 28 | 89 ± 8 | 86 ± 6 | 86 ± 6 |

**Patentansprüche**

1. Die Verwendung einer Verbindung der allgemeinen Formel

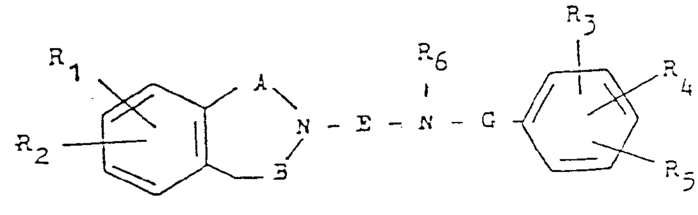

(I),

in der

A eine $-CH_2-CH_2-$, -CH=CH-,

$-NH-CO-$, $-CH_2-CO-$
5 5

oder

$R_7$
-C =N-Gruppe
5

in der $R_7$ eine Alkylgruppe mit 1 bis 3 Kohlenstoffatomen darstellt, und B die Methylen- oder Carbonylgruppe oder

A die -CO-CO-Gruppe und B die Methylengruppe,

E eine gegebenenfalls durch eine Alkylgruppe mit 1 bis 3 Kohlenstoffatomen substituierte Ethylen-, n-Propylen- oder n-Butylengruppe, eine durch eine Hydroxygruppe in 2-Stellung substituierte n-Propylengruppe oder eine durch eine Hydroxygruppe in 2- oder 3-Stellung substituierte n-Butylengruppe,

G eine gegebenenfalls durch eine Alkylgruppe mit 1 bis 3 Kohlenstoffatomen substituierte Methylen- oder Ethylengruppe,

$R_1$ und $R_2$, die gleich oder verschieden sein können, Hydroxygruppen, Alkyl-, Alkoxy- oder Phenylalkoxygruppen, in denen der Alkylteil jeweils 1 bis 3 Kohlenstoffatome enthalten kann, oder einer der Reste $R_1$ oder $R_2$ auch ein Wasserstoffatom oder $R_1$ zusammen mit $R_2$ eine Alkylendioxygruppe mit 1 oder 2 Kohlenstoffatomen,

$R_3$ und $R_4$, die gleich oder verschieden sein können, Wasserstoff- oder Halogenatome, Hydroxygruppen, Alkyl- oder Alkoxygruppen mit jeweils 1 bis 4 Kohlenstoffatomen, Trifluormethyl- oder Cyangruppen oder einer der Reste $R_3$ oder $R_4$ auch eine Nitrogruppe oder $R_3$ zusammen mit $R_4$ eine Alkylendioxygruppe mit 1 oder 2 Kohlenstoffatomen,

$R_5$ ein Wasserstoffatom, eine Alkyl-, Hydroxy-, Alkoxy-, Amino-, Alkylamino-, Dialkylamino-, Alkanoylamino-, Alkoxycarbonylamino- oder Bis(alkoxycarbonyl) aminogruppe, in denen der Alkylteil jeweils 1 bis 3 Kohlenstoffatome enthalten kann, oder eine durch eine Trifluormethylgruppe substituierte Methyalmino- oder Ethylaminogruppe, und

$R_5$ ein Wasserstoffatom, eine Alkyl-, Phenylalkyl, Alkanoyl- oder Alkoxycarbonylgruppe, in denen der Alkylteil jeweils 1 bis 3 Kohlenstoffatome enthalten kann, oder eine Alkylengruppe mit 3 bis 5 Kohlenstoffatomen bedeuten, und deren Säureadditionssalzen, insbesondere deren physiologisch verträgliche Säureadditionssalze mit anorganischen oder organischen Säuren, zur Herstellung eines pharmazeutischen Präparates zur Behandlung von Herzinsuffizienz.

**2.** Die Verwendung gemäß Anspruch 1, dadurch gekennzeichnet, daß man 1,3,4,5-tetrahydro-7,8-dimethoxy-3-[3-[[2-(3,4-dimethoxyphenyl)ethyl]methyl]amino]propyl]-2H-3-benzazepin-2-on oder eine seiner physiologisch verträglichen Säureadditionssalze verwendet.

**3.** Die Verwendung gemäß Anspruch 2, dadurch gekennzeichnet, daß als Säureadditionssalz das Hydrogenchlorid verwendet wird.

## Claims

1. The use of a compound of general formula

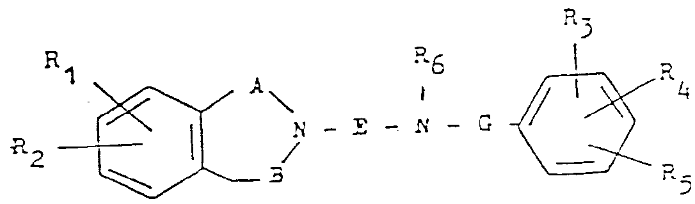 (I),

wherein

A represents a $-CH_2-CH_2-$, -CH=CH-,

$-NH-CO-$, $-CH_2-CO-$
5 5

or

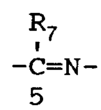

group wherein $R_7$ represents an alkyl group having 1 to 3 carbon atoms, and B represents a methylene or carbonyl group or

A represents a -CO-CO- group and B represents a methylene group,

E represents an ethylene, n-propylene or n-butylene group optionally substituted by an alkyl group with 1 to 3 carbon atoms, an n-propylene group substituted by a hydroxy group in the 2-position or an n-butylene group substituted by a hydroxy group in the 2- or 3-position,

G represents a methylene or ethylene group optionally substituted by an alkyl group with 1 to 3 carbon atoms,

$R_1$ and $R_2$, which may be identical or different, represent hydroxy groups, alkyl, alkoxy or phenylalkoxy groups in which the alkyl moiety may contain from 1 to 3 carbon atoms, or one of the groups $R_1$ or $R_2$ may also represent a hydrogen atom or $R_1$ together with $R_2$ represents an alkylenedioxy group with 1 or 2 carbon atoms,

$R_3$ and $R_4$, which may be identical or different, represent hydrogen or halogen atoms, hydroxy groups, alkyl or alkoxy groups each having 1 to 4 carbon atoms, trifluoromethyl or cyano groups or one of the groups $R_3$ or $R_4$ may also represent a nitro group or $R_3$ together with $R_4$ represents an alkylenedioxy group with 1 or 2 carbon atoms,

$R_5$ represents a hydrogen atom, an alkyl, hydroxy, alkoxy, amino, alkylamino, dialkylamino, alkanoylamino, alkoxycarbonylamino or bis(alkoxycarbonyl)amino group wherein the alkyl part may contain from 1 to 3 carbon atoms, or a methylamino or ethylamino group substituted by a trifluoromethyl group, and

$R_5$ represents a hydrogen atom, an alkyl, phenylalkyl, alkanoyl or alkoxycarbonyl group in which the alkyl moiety may contain from 1 to 3 carbon atoms, or an alkylene group with 3 to 5 carbon atoms,

and the acid addition salts thereof, particularly the physiologically acceptable acid addition salts thereof with inorganic or organic acids, for the manufacture of a pharmaceutical preparation for the treatment of cardiac insufficiency.

2. The use as claimed in claim 1, characterised in that 1,3,4,5-tetrahydro-7,8-dimethoxy-3-[3-[[2-(3,4-dimethoxyphenyl) ethyl]methyl]amino]propyl]-2H-3-benzazepin-2-one or one of the physiologically acceptable acid addition salts thereof is used.

3. The use as claimed in claim 2, characterised in that the hydrochloride is used as the acid addition salt.

## Revendications

1. Utilisation d'un composé de formule générale

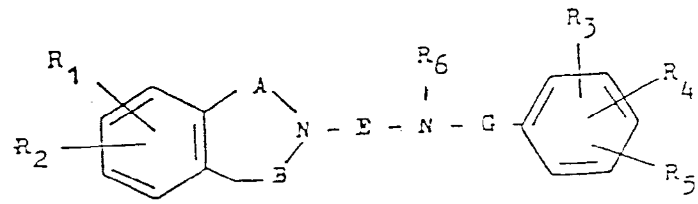

(I),

dans laquelle

A représente un groupe $-CH_2-CH_2-$, $-CH=CH-$,

$-NH-CO-$,

5

$-CH_2-CO-$

5

ou

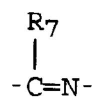

5

où $R_7$ représente un groupe alkyle de 1 à 3 atomes de carbone, et B représente le groupe méthylène ou carbonyle ou

A représente le groupe -CO-CO- et B représente le groupe méthylène,

E représente un groupe éthylène, n-propylène ou n-butylène éventuellement substitué par un groupe alkyle de 1 à 3 atomes de carbone, un groupe n-propylène substitué par un groupe hydroxyle en position 2 ou un groupe n-butylène substitué par un groupe hydroxyle en position 2 ou 3,

G représente un groupe méthylène ou éthylène éventuellement substitué par un groupe alkyle de 1 à 3 atomes de carbone,

$R_1$ et $R_2$, qui peuvent être identiques ou différents, représentent des groupes hydroxyle, alkyle, alcoxy ou phénylalcoxy dans lesquels la partie alkyle peut contenir dans chaque cas 1 à 3 atomes de carbone, ou bien l'un des restes $R_1$ et $R_2$ peut aussi représenter un atome d'hydrogène ou bien $R_1$ représente avec $R_2$ un groupe alkylènedioxy de 1 ou 2 atomes de carbone,

$R_3$ et $R_4$, qui peuvent être identiques ou différents, représentent des atomes d'hydrogène ou d'halogène, des groupes hydroxyle, des groupes alkyle ou alcoxy contenant chacun 1 à 4 atomes de carbone, des groupes trifluorométhyle ou cyano ou bien l'un des restes $R_3$ et $R_4$ représente aussi un groupe nitro ou bien $R_3$ représente avec $R_4$ un groupe alkylènedioxy de 1 ou 2 atomes de carbone,

$R_5$ représente un atome d'hydrogène, un groupe alkyle, hydroxyle, alcoxy, amino, alkylamino, dialkylamino, alcanoylamino, alcoxycarbonylamino ou bis(alcoxycarbonyl)amino, dans lesquels la partie alkyle peut contenir dans chaque cas 1 à 3 atomes de carbone, ou bien un groupe méthylamino ou éthylamino substitué par un groupe trifluorométhyle, et

$R_5$ représente un atome d'hydrogène, un groupe alkyle, phénylalkyle, alcanoyle ou alcoxycarbonyle, dans lesquels la partie alkyle peut contenir dans chaque cas 1 à 3 atomes de carbone, ou bien un groupe alkylène de 3 à 5 atomes de carbone, et de ses sels d'addition d'acide, en particulier de ses sels d'addition d'acide acceptables du point de vue physiologique avec des acides inorganiques ou organiques, pour l'obtention d'une préparation pharmaceutique pour le traitement de l'insuffisance cardiaque.

2. Utilisation selon la revendication 1, caractérisée en ce que l'on utilise la 1,3,4,5-tétrahydro-7,8-diméthoxy-3-[3-[[2-(3,4-diméthoxyphényl)éthyl]méthyl]amino]propyl]-2H-3-benzazépine-2-one ou l'un de ses sels d'addition d'acide acceptables du point de vue physiologique.

3. Utilisation selon la revendication 2, caractérisée en ce que l'on utilise comme sel d'addition d'acide le chlorhydrate.

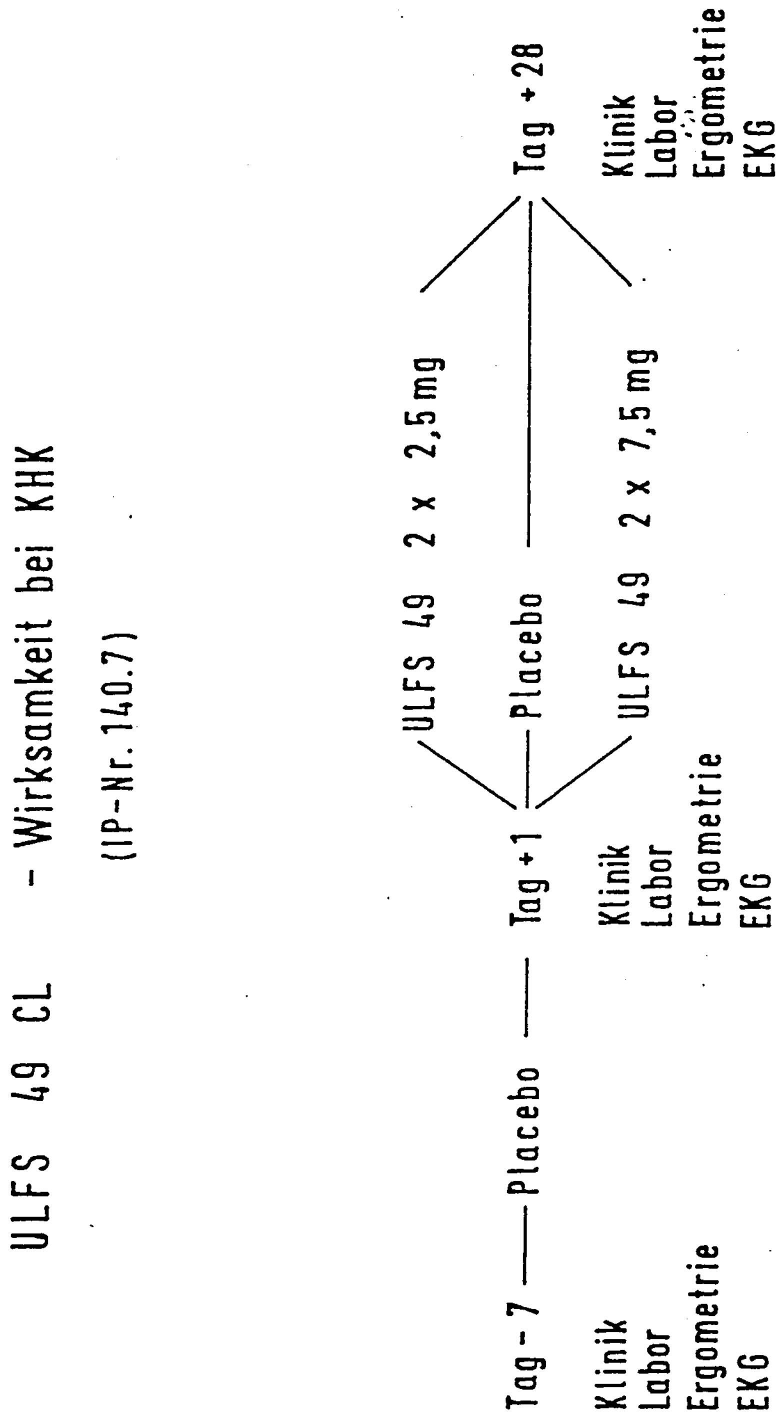
ULFS 49 CL - Wirksamkeit bei KHK
(IP-Nr. 140.7)
Tag - 7
Klinik
Labor
Ergometrie
EKG
Placebo
Tag + 1
Klinik
Labor
Ergometrie
EKG
ULFS 49 2 x 2,5 mg
Placebo
ULFS 49 2 x 7,5 mg
Tag + 28
Klinik
Labor
Ergometrie
EKG

Fig. 1

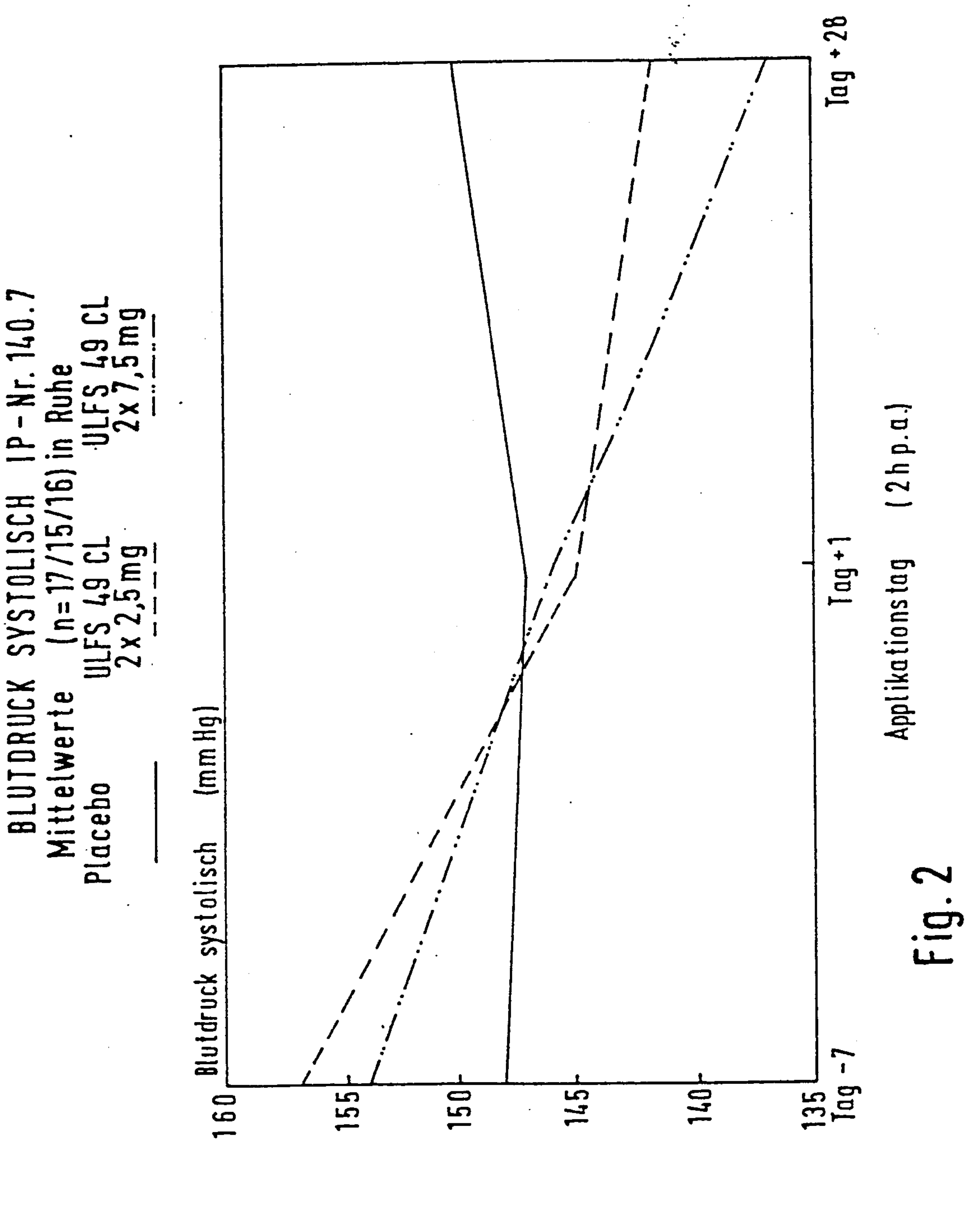
BLUTDRUCK SYSTOLISCH IP-Nr. 140.7
Mittelwerte (n=17/15/16) in Ruhe
Placebo
ULFS 49 CL
2x 2,5 mg
ULFS 49 CL
2x 7,5 mg
Blutdruck systolisch (mm Hg)
160
155
150
145
140
135
Tag -7
Tag +1
Tag +28
Applikationstag (2 h p.a.)

Fig. 2

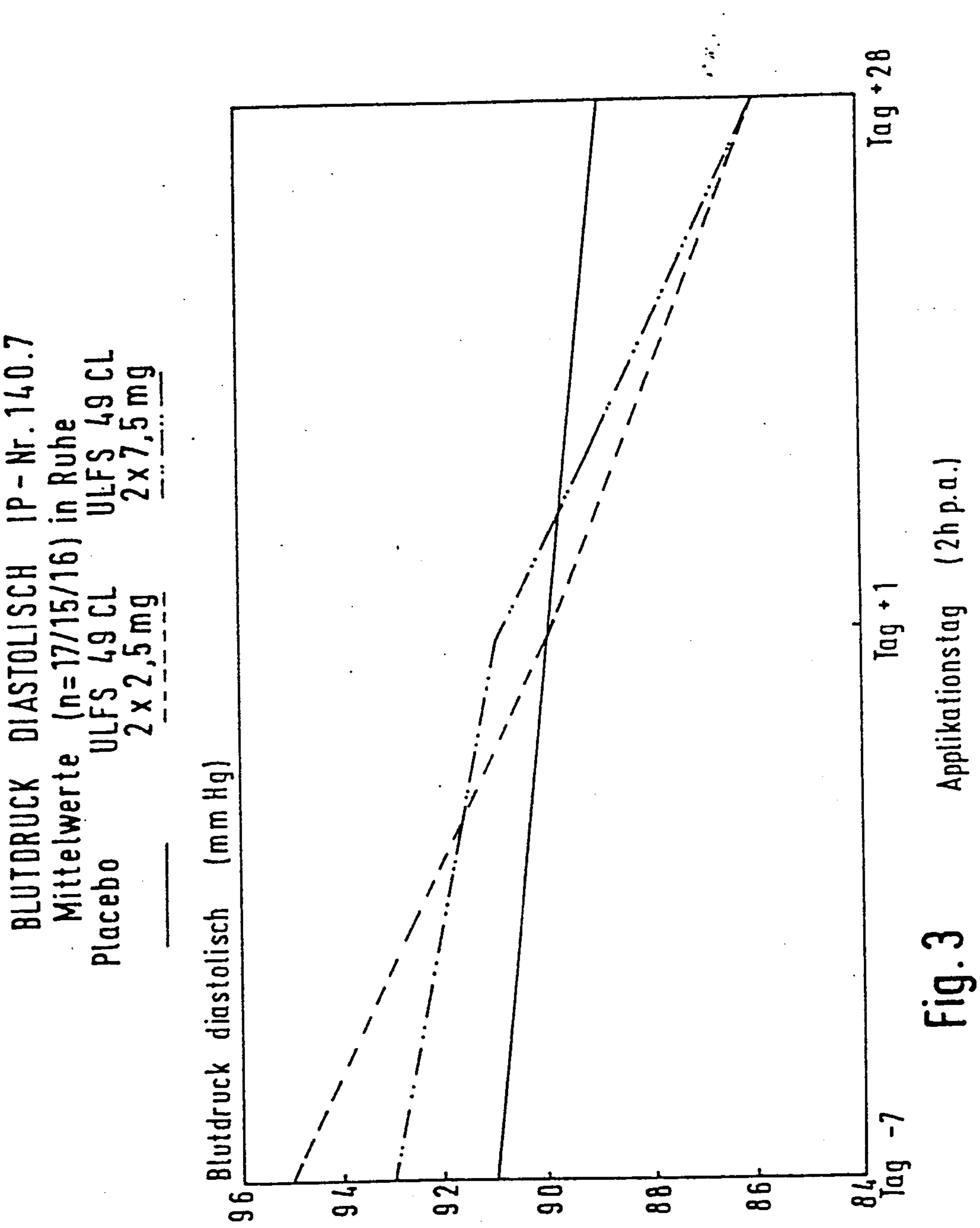

BLUTDRUCK DIASTOLISCH IP - Nr. 14 0.7
Mittelwerte (n=17/15/16) in Ruhe
Placebo
ULFS 49 CL
2 x 2,5 mg
ULFS 49 CL
2 x 7,5 mg
Blutdruck diastolisch (mm Hg)
96
94
92
90
88
86
84
Tag -7
Tag +1
Tag +28
Applikationstag (2h p.a.)


Fig. 3